Europäisches Patentamt

European Patent Office

Office européen des brevets

(11) Numéro de publication : **0 172 070**
**B1**

(12)

# FASCICULE DE BREVET EUROPÉEN

(45) Date de publication du fascicule du brevet :
**30.09.87**

(51) Int. Cl.⁴ : **C 02 F   3/28**

(21) Numéro de dépôt : **85401418.0**

(22) Date de dépôt : **11.07.85**

(54) **Digesteur et procédé de digestion anaérobie le mettant en oeuvre.**

(30) Priorité : **20.07.84 FR 8411535**

(43) Date de publication de la demande :
**19.02.86 Bulletin 86/08**

(45) Mention de la délivrance du brevet :
**30.09.87 Bulletin 87/40**

(84) Etats contractants désignés :
**AT BE CH DE FR GB IT LI LU NL SE**

(56) Documents cités :
**DE-C-   136 164**
**FR-A-   467 652**
**US-A- 1 641 800**

(73) Titulaire : **Groupement d'Intérêt Economique dit :**
**BIOMAGAZ**
**62, rue Jeanne d'Arc**
**F-75013 Paris (FR)**

(72) Inventeur : **Aubart, Christian**
**1, rue des Ormes Parc d'Entremont**
**F-68170 Rixheim (FR)**
Inventeur : **Peschel, Claude**
**38, Avenue Gleyze Vieille**
**F-31520 Ramonville-Saint-Agné (FR)**
Inventeur : **Bully, François**
**12, rue Brignais Schweighouse**
**F-68520 Burnhaupt-le-Haut (FR)**
Inventeur : **Festino, Corinne**
**67, Avenue de Colmar**
**F-68100 Mulhouse (FR)**

(74) Mandataire : **Bourgognon, Jean-Marie et al**
**Cabinet Flechner 22, Avenue de Friedland**
**F-75008 Paris (FR)**

EP 0 172 070 B1

Jouve, 18, rue St-Denis, 75001 Paris, France

# 0 172 070

## Description

L'invention concerne des digesteurs et des procédés de digestion anaérobie de matières organiques. Elle s'applique notamment à la digestion de déchets organiques et végétaux d'effluents d'origine agricole et agro-alimentaire, tels que le lisier de porcs, le lisier de bovins, un mélange d'eau ou de lisier et de fientes de volailles, un mélange d'eau ou de lisier et de crottes de lapins, des déchets d'abattoirs, et autres déchets semblables, mais aussi, d'une manière générale, provenant d'effluents liquides contenant des matières organiques solides en suspension, à raison notamment de 1 à 10 % environ en poids.

On connaît déjà un digesteur constitué d'une enceinte étanche à l'air. Un conduit d'entrée de la matière à digérer débouche dans le bas de l'enceinte. Un conduit de sortie de la matière digérée débouche dans l'enceinte, à un niveau plus élevé que celui de l'embouchure du conduit d'entrée et à distance de celle-ci, suivant une direction horizontale. Une conduite de sortie du gaz débouche au sommet de l'enceinte qui est munie de moyens de thermostatisation. On a également déjà proposé, à la demande de brevet européen EP-A 70 245, de prévoir une cloison mobile dans le digesteur destinée à racler des croûtes.

Dans des digesteurs de ce type, on charge périodiquement la matière à digérer et l'on procède à la fermentation anaérobie à une température constante, habituellement de 35 à 37 °C, permettant le développement d'une population de bactéries mésophiles, et en agitant le contenu du digesteur par des moyens d'agitation afin de mieux répartir les calories, d'éviter l'accumulation des matériaux du déchet organique qui sédimente et la formation d'une croûte à la surface du liquide. Le biogaz est stocké dans un gazomètre qui communique avec le conduit de sortie du gaz, par l'intermédiaire d'une vanne, les autres conduits d'entrée et de sortie étant également munis de vannes.

On juge des performances d'un digesteur par les variations de teneur en matières sèches (M.S.), de teneur en matière volatile ou organique (M.O.), par la variation de la demande chimique en oxygène (D.C.O.), du produit avant et après digestion, par la production journalière de biogaz et par la composition de ce biogaz.

L'invention vise un digesteur et un procédé de digestion anaérobie de matières organiques nouveaux qui permettent, d'une manière simple, d'améliorer les performances mentionnées ci-dessus.

Le digesteur suivant l'invention se caractérise par une première cloison issue du fond de l'enceinte et interposée, suivant ladite direction horizontale, entre les embouchures des conduits d'entrée et de sortie en subdivisant l'enceinte en un premier compartiment et en une chambre ne communiquant entre eux qu'au-dessus du bord de la cloison qui est à un niveau intermédiaire entre les deux embouchures, et par au moins une deuxième cloison issue du fond de l'enceinte et interposée suivant ladite direction horizontale entre la première cloison et l'embouchure du conduit de sortie, en subdivisant la chambre en des deuxième et troisième compartiments ne communiquant entre eux qu'au-dessus du bord de la deuxième cloison qui est à un niveau inférieur à celui de la première cloison.

Le procédé de digestion anaérobie suivant l'invention consiste à charger périodiquement un digesteur suivant l'invention d'une suspension de matières organiques et à laisser le contenu du digesteur se décanter dans les intervalles de temps entre les chargements.

La combinaison d'au moins deux cloisons, définissant donc un deuxième compartiment intermédiaire entre le premier dans lequel débouche le conduit d'entrée, et un compartiment subséquent dans lequel débouche le conduit de sortie, et de la décantation dans les intervalles de temps entre les chargements, permet d'obtenir des performances nettement supérieures à celles des digesteurs antérieurs. Bien loin de chercher à homogénéiser le contenu du digesteur par un dispositif d'agitation, ou par une cloison racleuse, on y définit un deuxième compartiment dans lequel la décantation va s'effectuer au mieux dans les intervalles de temps entre les chargements, mais aussi en dehors de ces intervalles, puisque le contenu de ce compartiment est agité aussi peu que possible pendant les chargements, la chasse produite par le chargement dans le premier compartiment ne se transmettant pas au fond du deuxième compartiment et les tourbillons et agitations provoqués par la sortie de l'effluent dans le dernier compartiment ne se transmettant pas non plus au deuxième compartiment intermédiaire qui en est protégé par les cloisons. Et cette technique de décantation, qui va directement à l'encontre de la technique antérieure qui s'efforçait de maintenir une agitation, donne des résultats bien supérieurs, d'autant que, pendant les intervalles de temps, la décantation s'effectue dans tous les compartiments.

Lors des chargements, il convient que la partie de la suspension la moins lourde, se trouvant en haut des compartiments, passe dans le compartiment suivant ou soit évacuée, sans pour autant que la partie lourde se trouvant plus bas soit entraînée. On a obtenu, à cet effet, des résultats satisfaisants quand le bord libre de la première cloison est à un niveau supérieur à la demi-hauteur de l'enceinte, et quand le bord libre de la deuxième cloison est à un niveau compris entre les deux-tiers et le tiers de la hauteur de l'enceinte. Pour la même raison, le volume déterminé entre la paroi de l'enceinte et la première cloison représente, de préférence, de 1,1 à 1,4 fois celui délimité entre la première cloison et une deuxième cloison.

Il est avantageux de prévoir une troisième cloison moins haute que la deuxième et subdivisant le troisième compartiment en un troisième compartiment proprement dit et en un quatrième compartiment. De préférence, le bord libre de la troisième cloison est à un niveau compris entre les 2/5 et le 1/4 de la

2

**0 172 070**

hauteur de l'enceinte et le volume délimité entre la première cloison et la deuxième cloison représente de 2,5 à 1,5 fois celui délimité entre la deuxième cloison et la troisième cloison.

Suivant une variante de réalisation, ladite direction horizontale est circulaire, l'enceinte est cylindrique et les bords des cloisons s'étendent suivant des rayons de la section droite de l'enceinte.

Il convient que les intervalles de temps entre les chargements soient suffisants pour que la décantation s'effectue correctement. La durée d'un intervalle de temps est avantageusement comprise entre 2 et 24 heures et la durée d'un chargement est de préférence inférieure au 1/12e de la durée d'un intervalle entre les chargements.

Le processus de démarrage du digesteur consiste à y introduire des bactéries de fermentation des matières organiques. Ce sont essentiellement des bactéries acidogènes, telles que Bacillus, Arthrobacter, Staphilococcus, Pseudomonas, Cellulomonas, Corynebacterium, Erwinia, Eubacterium, et des bactéries méthanogènes, telles que Methanobacterium formicium, Methanobacterium arborphilicum, Methanobacterium mobile, Methanobacterium thermoautotrophicum, Methanospirillum hungatii, Methanosarcina barkeri, Methanococcus vanielli.

On peut introduire ces bactéries en introduisant, dans le digesteur, un effluent déjà digéré et en laissant les bactéries s'adapter et se développer pendant un temps suffisant dans le digesteur.

Au dessin annexé, donné uniquement à titre d'exemple :

la figure 1 est une vue en coupe schématique d'un digesteur de laboratoire suivant l'invention,

la figure 2 est une vue en coupe verticale d'une variante de digesteur suivant l'invention,

la figure 3 est une vue en coupe, suivant un plan vertical perpendiculaire au plan de coupe de la figure 2,

la figure 4 est une vue en coupe, suivant la ligne IV-IV de la figure 2,

la figure 5 est une vue en coupe suivant la ligne V-V de la figure 2,

la figure 6 est une vue en coupe suivant la ligne VI-VI de la figure 2, et

les figures 7 à 17 sont des graphiques illustrant les performances du digesteur de la figure 1.

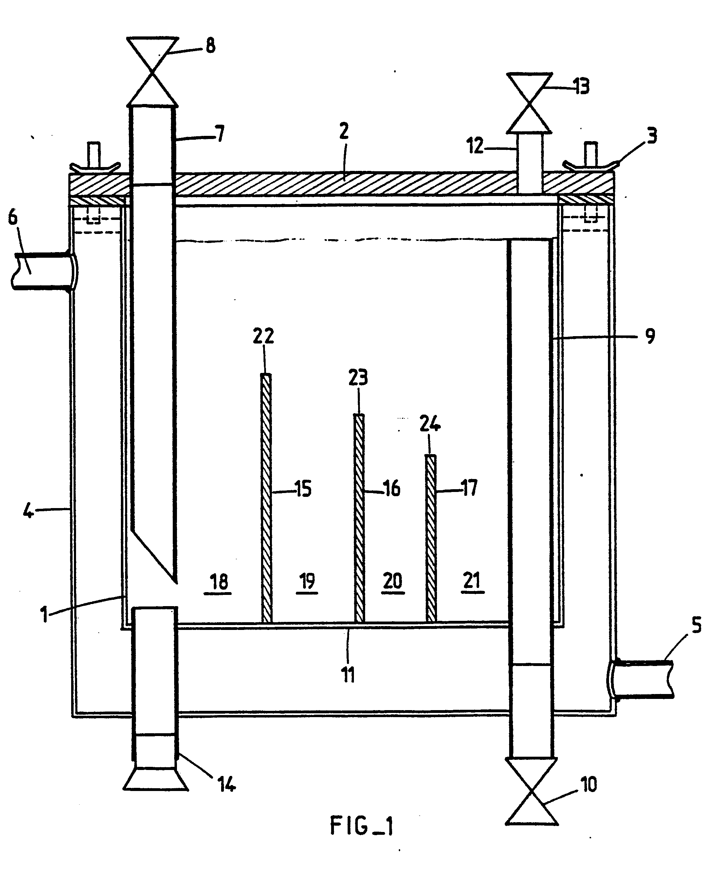

Le digesteur représenté à la figure 1 comprend une enceinte 1 cylindrique fermée de manière étanche à l'air par un couvercle 2, au moyen de vis papillon 3. L'enceinte 1 est entourée d'une enveloppe 4 munie d'un raccord 5 d'entrée communiquant avec une source d'eau chaude, à 37 °C, et d'un raccord 6 de sortie. L'enveloppe 4 emplie d'eau chaude à 37 °C sert à maintenir le digesteur à une température constante.

Un conduit 7 d'entrée, avec vanne 8, débouche, en traversant le couvercle 2, à l'intérieur de l'enceinte 1 dans le bas de celle-ci. Le conduit 7 vertical est disposé au voisinage de la paroi cylindrique de l'enceinte 1. Un conduit 9 de sortie, avec vanne 10, débouche, en traversant le fond 11, dans l'enceinte 1 à la partie supérieure de celle-ci. Les conduits 7 et 9 sont diamétralement opposés. Une conduite 12, avec vanne 13, de sortie du gaz débouche au sommet de l'enceinte 1, en traversant le couvercle 2. Une sonde de température 14 permet de connaître la température à l'intérieur de l'enceinte 1.

Du fond 11 de l'enceinte 1 partent trois cloisons 15, 16 et 17 s'étendant perpendiculairement au diamètre sur lequel se trouvent les deux conduits 7 et 9. Ces cloisons subdivisent l'enceinte 1 en des premier, deuxième, troisième et quatrième compartiments 18, 19, 20 et 21 se faisant suite du conduit 7 au conduit 9. La hauteur de l'enceinte du fond 11 au couvercle 2 est de 20 cm. Le point le plus bas du conduit 7 est à 2 cm au-dessus du fond. Le conduit 9 débouche à 1,5 cm en dessous du couvercle 2. Le bord supérieur 22 libre de la cloison 15 est à 12 cm au-dessus du fond 11. Le bord supérieur 23 de la cloison 16 est à 10 cm au-dessus du fond 11 et le bord supérieur 24 de la cloison 17 est à 8 cm au-dessus du fond 11. La cloison 15 est distante de la paroi latérale la plus proche de la cuve 1 de 6,5 cm et de la cloison 16 de 4 cm. La cloison 17 est distante de la cloison 16 de 3 cm. Le volume du compartiment 18 jusqu'au niveau du bord supérieur 22 est de 1,080 dm³. Celui du compartiment 19 jusqu'au niveau du bord 23 est de 0,807 dm³, celui du compartiment 20 jusqu'au niveau de la cloison 24 de 0,474 dm³ et celui du compartiment 21 jusqu'au niveau de la cloison 24 de 0,616 dm³.

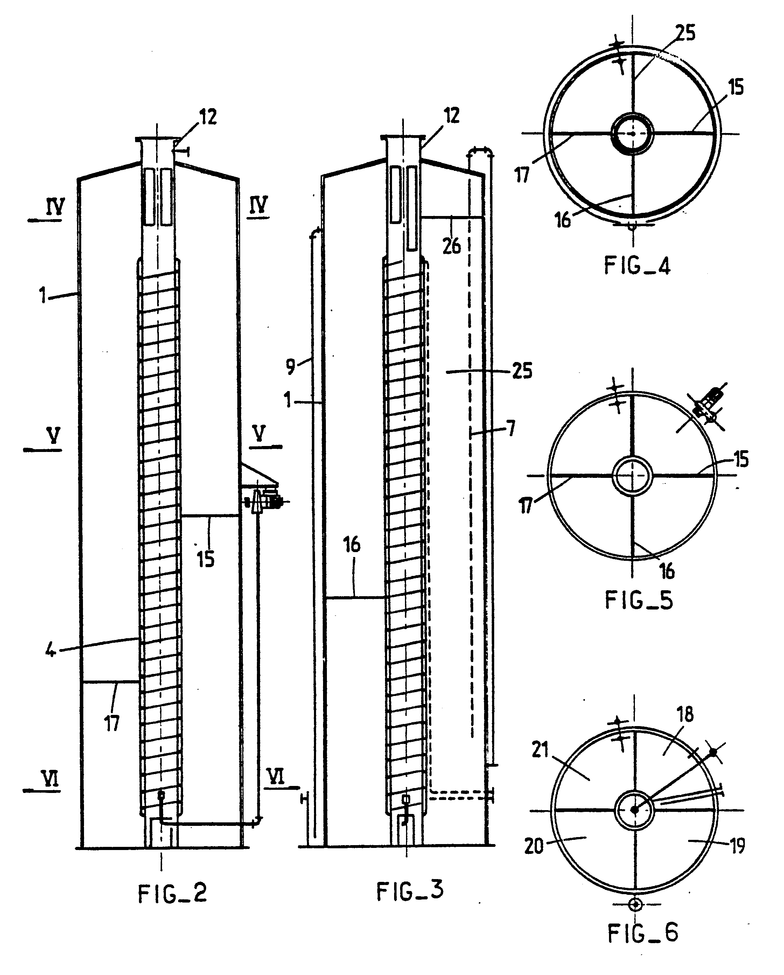

Aux figures 2 à 6, est représentée une variante du digesteur suivant l'invention. On y a repéré par des signes de référence identiques les éléments qui ont les mêmes fonctions que dans le digesteur de la figure 1. On notera, à cet effet, que la chemise 4 de thermostatisation est remplacée par un échangeur de chaleur central. Les cloisons 15, 16, 17 affectent la forme de cloisons radiales verticales et sont complétées, de manière à former quatre compartiments 18, 19, 20, 21, par une cloison 25 radiale verticale dont le bord supérieur 26 est à un niveau supérieur à l'embouchure du conduit 9 dans l'enceinte 1. Les cloisons 25, 15, 16 et 17 délimitent ainsi les compartiments 18, 19, 20 et 21. Si, par convention, on fixe à 100 la hauteur de la cloison 25, la cloison 15 a une hauteur de 50, la cloison 16 une hauteur de 35 à 40 et la cloison 17 une hauteur de 25.

Dans les deux variantes, les particules les plus lourdes demeurent plus longtemps dans le compartiment 18 que la moyenne de l'effluent, parce que, en raison de leur densité, elles se trouvent entraînées vers le fond du compartiment 18 et, lors d'un chargement, ne sont pas repoussées au-dessus du bord 22 de la cloison 15. Ce temps de séjour plus long que le temps de séjour moyen de l'effluent permet d'assurer une dégradation plus complète des particules les plus grosses et ce n'est que lorsqu'elles diminuent de poids que ces particules allégées ont tendance à reprendre le trajet du courant moyen. Les mêmes phénomènes se produisent dans les autres compartiments et tout particulièrement dans les compartiments intermédiaires 19 et 20, puisque dans ceux-ci les particules les plus grosses, qui

3

se trouvent au fond, ne sont pas refoulées vers le haut par l'arrivée d'un volume nouveau d'effluent, puisque le passage de l'effluent, lors du chargement, s'effectue dans l'espace se trouvant au-dessus des compartiments 19 et 20. Les exemples suivants illustrent l'invention.

Exemple 1

On envoie 3,0 litres d'effluent frais et 3,0 litres d'effluent digéré de lisier de porc provenant d'un autre digesteur en fonctionnement, par le conduit 7, dans le digesteur de la figure 1. On n'alimente plus le digesteur pendant 4 jours, puis pendant une semaine on introduit 400 ml par jour de lisier frais. Après cette période d'adaptation et de développement des bactéries, on introduit 600 ml par jour de lisier dans le digesteur, fonctionnant ainsi en régime stable, et cela pendant 52 semaines. On maintient la température du digesteur à 37 °C en faisant circuler de l'eau chaude dans la chemise 4, par l'intermédiaire des raccords 5 et 6.

Les caractéristiques du produit introduit dans le digesteur, à savoir pH, MS, MVO et DCO (sur le produit brut) et MS et MVO (du surnageant) sont déterminées sur un échantillon moyen une fois par semaine. Ces mêmes caractéristiques sont déterminées également sur l'effluent du digesteur sur un échantillon moyen une fois par quinzaine. On mesure la production de biogaz chaque jour. La composition du biogaz est déterminée bimensuellement en déterminant la teneur en $CH_4$, en $CO_2$ et en $H_2S$.

On effectue la mesure du pH sur des échantillons aussi homogènes que possible, en agitant juste avant la mesure, à l'aide d'un pHmètre digital-Bioblock Scientific 87 (35).

La matière sèche (M.S.), qui est l'ensemble des substances organiques et minérales en solution ou en suspension dans le lisier, est déterminée par évaporation de l'eau de 10 ml de substrat en opérant dans une capsule en porcelaine à 75 °C pendant 36 heures. Cette teneur en matière sèche est exprimée en gramme/litre.

La matière volatile ou organique (M.O.) est exprimée en calculant le pourcentage de matière organique volatile par rapport à la matière totale, déterminée par la différence entre la matière sèche obtenue par évaporation à 75 °C pendant 36 heures, et les résidus de cendres obtenus par calcination à 600 °C pendant 1 heure (exprimée en % par rapport à la M.S.).

Sur le surnageant, on effectue des mesures de la M.S. et de la M.O. et, dans certains cas, de la D.C.O. (demande chimique en oxygène). Cette mesure s'effectue en centrifugeant le lisier brut à 12 000 tours/minute pendant 15 minutes et en procédant, pour la M.S. et la M.O., de la même manière que ci-dessus.

Pour la demande chimique en oxygène, qui indique la consommation en oxygène d'une eau pour l'oxydation de presque toutes les substances organiques solubles dans l'eau, et que l'on exprime en milligrammes d'oxygène par litre de liquide, la technique utilisée est celle définie par la norme AFNOR N° T.90.101 (DEGREMONT, 1978) pour les eaux résiduaires. Mais en raison de la nature très particulière des lisiers, qui ont beaucoup de matière sèche en suspension, la prise d'essai est de 25 ml au lieu de 50 ml, et l'échantillon subit au préalable une dilution de 2 ml dans 200 pour des lisiers bruts non digérés à 4 % de M.S. et de 4 ml dans 200 pour des lisiers digérés dont le lisier brut était de 4 % de M.S.

Les concentrations de $CH_4$, $CO_2$ et $H_2S$ dans le biogaz sont mesurées par chromatographie en phase gazeuse à catharomètre (modèle 3 000, GIRDEL). La colonne, de 400 cm de longueur, est remplie de Porapack Q.

Les caractéristiques du produit d'entrée ont été regroupées en cinq groupes de valeur :

| | |
|---|---|
| 1 | 2,06 % < MS < 2,56 % |
| 2 | 2,63 % < MS < 3,19 % |
| 3 | 2,63 % < MS < 3,59 % |
| 4 | 3,25 % < MS < 3,59 % |
| 5 | 3,98 % < MS < 4,22 % |

Le groupe 3 correspond à la moyenne des groupes 2 et 4.
Les résultats obtenus sont consignés dans les tableaux 1 et 2 suivants.

(Voir tableaux page 5 et s.)

4

0 172 070

## Tableau 1

### Epuration sur le produit brut

|  | 1 | | 2 | | 3 | | 4 | | 5 | |
|---|---|---|---|---|---|---|---|---|---|---|
|  | $2,06\% < MS < 2,56\%$ | | $2,63\% < MS < 3,19\%$ | | $2,63\% < MS < 3,59\%$ | | $3,25\% < MS < 3,59\%$ | | $3,98\% < MS < 4,22\%$ | |
| $\overline{MS}$ % | 2,28 | $\sigma:0,16$ | 2,99 | $\sigma:0,19$ | 3,13 | $\sigma:0,27$ | 3,44 | $\sigma:0,15$ | 4,12 | $\sigma:0,11$ |
| $\overline{MVO}$ % | 1,55 | $\sigma:0,10$ | 2,00 | $\sigma:0,10$ | 2,08 | $\sigma:0,15$ | 2,26 | $\sigma:0,07$ | 2,81 | $\sigma:0,10$ |
| $\overline{DCO}$ mg $O_2/l$ | 27721 | $\sigma:3395$ | 33995 | $\sigma:2739$ | 34669 | $\sigma:3162$ | 36247 | $\sigma:3708$ | 43964 | $\sigma:10,11$ |
| $\overline{\Delta MS}$ % | 48,1 | $\sigma:12,3$ | 55,1 | $\sigma:7,4$ | 54,1 | $\sigma:9,0$ | 48,5 | $\sigma:10,9$ | 35,6 | $\sigma:15,3$ |
| $\overline{\Delta MVO}$ % | 57,7 | $\sigma:12,9$ | 64,7 | $\sigma:6,8$ | 61,7 | $\sigma:9,6$ | 55,8 | $\sigma:11,9$ | 46,4 | $\sigma:12,8$ |
| $\overline{\Delta DCO}$ % | 70,2 | $\sigma: 5,7$ | 72,8 | $\sigma:3,9$ | 70,1 | $\sigma:6,6$ | 64,7 | $\sigma: 7,8$ | 55,2 | $\sigma:6,8$ |

## Tableau 2

### Epuration sur le surnageant de centrifugation

|  | 1 | | 2 | | 3 | | 4 | | 5 | |
|---|---|---|---|---|---|---|---|---|---|---|
| $\overline{MS}_B$ % | 0,83 | $\sigma:0,13$ | 1,07 | $\sigma:0,12$ | 1,10 | $\sigma:0,12$ | 1,16 | $\sigma:0,12$ | 1,26 | $\sigma:0,04$ |
| $\overline{MVO}_B$ % | 0,46 | $\sigma:0,09$ | 0,57 | $\sigma:0,08$ | 0,59 | $\sigma:0,07$ | 0,61 | $\sigma:0,05$ | 0,66 | $\sigma:0,05$ |
| $\overline{\Delta MS}_B$ % | 35,3 | $\sigma:6,4$ | 34,9 | $\sigma:5,4$ | 32,7 | $\sigma:6,7$ | 28,2 | $\sigma:7,1$ | 33,9 | $\sigma:1,2$ |
| $\overline{\Delta MVO}_B$ % | 52,3 | $\sigma:9,4$ | 54,0 | $\sigma:5,9$ | 49,9 | $\sigma:9,2$ | 41,8 | $\sigma:9,6$ | 53,7 | $\sigma:5,9$ |

Tableau 3

Production de biogaz et rendements biologiques

| | 1 | | 2 | | 3 | | 4 | | 5 | |
|---|---|---|---|---|---|---|---|---|---|---|
| $\overline{MS}$ % | 2,28 | σ:0,16 | 2,99 | σ:0,19 | 3,13 | σ:0,27 | 3,44 | σ:0,15 | 4,12 | σ:0,11 |
| $\overline{MVO}$ % | 1,55 | σ:0,10 | 2,00 | σ:0,10 | 2,08 | σ:0,15 | 2,26 | σ:0,07 | 2,81 | σ:0,10 |
| $\overline{DCO}$ mg $O_2$/l | 27721 | σ:3395 | 33995 | σ:2739 | 34669 | σ:3162 | 36247 | σ:3708 | 43964 | σ:1011 |
| $\overline{RT}$ biogaz/ldig/j | 0,91 | σ:0,13 | 1,13 | σ:0,13 | 1,14 | σ:0,14 | 1,15 | σ:0,15 | 1,45 | σ:0,11 |
| % $\overline{CH_4}$ | 69,7 | σ:3,4 | 69,5 | σ:2,0 | 69,4 | σ:1,9 | 69,1 | σ:1,7 | 68,9 | σ:0,4 |
| $\overline{RB}$ $CH_4$/kg MS | 283 | σ:41 | 269 | σ:35 | 257 | σ:37 | 232 | σ:29 | 241 | σ:16 |
| biogaz/kg MS | 405 | σ:47 | 387 | σ:50 | 371 | σ:53 | 336 | σ:42 | 351 | σ:24 |
| $\overline{RB}$ $CH_4$/kg MVO | 416 | σ:70 | 400 | σ:58 | 385 | σ:59 | 353 | σ:51 | 352 | σ:24 |
| biogaz/kg MVO | 594 | σ:80 | 576 | σ:84 | 556 | σ:85 | 510 | σ:71 | 513 | σ:35 |
| $\overline{RB}$ $CH_4$/kg DCO | 232 | σ:34 | 232 | σ:22 | 228 | σ:23 | 221 | σ:24 | 244 | σ:33 |
| biogaz/kg DCO | 332 | σ:42 | 331 | σ:36 | 329 | σ:36 | 320 | σ:39 | 330 | σ:29 |

En résumé les abattements de MS, MVO, DCO obtenus sur un lisier ayant une teneur en MS de 2 à 2,5 % sont respectivement de 45 à 55 %, 50 à 70 %, 60 à 75 % avec un temps de rétention de 10 jours.

Le rendement biologique est de 400 litres de biogaz/kg MS soit 30 % de plus qu'avec un système de digesteur infiniment mélangé.

Pour un lisier ayant une teneur en MS de 3 à 3,5 %, les abattements de MS, MVO et DCO sont respectivement de 50 à 60 %, 60 à 75 %, 65 à 80 %. Le rendement biologique est de 380 litres de biogaz/kg MS.

Les résultats obtenus avec un lisier à 4 % de MS sont 30 à 50 % d'abattement de MS, 40 à 40 % d'abattement de MVO, 45 à 60 % d'abattement de DCO. Le rendement biologique est de 350 litres de biogaz/kg MS.

Pour une porcherie produisant 20 m$^3$ de lisier à 3,5 % de MS par jour, la production de biogaz attendue par digestion anaérobie de ce substrat dans un digesteur à rétention de solides avec un temps de séjour du produit de 10 jours est de 260 m$^3$ de biogaz/jour à 69,4 % de $CH_4$.

Le produit digéré se caractérise par une DCO inférieure à la DCO initiale de 65 à 70 %.

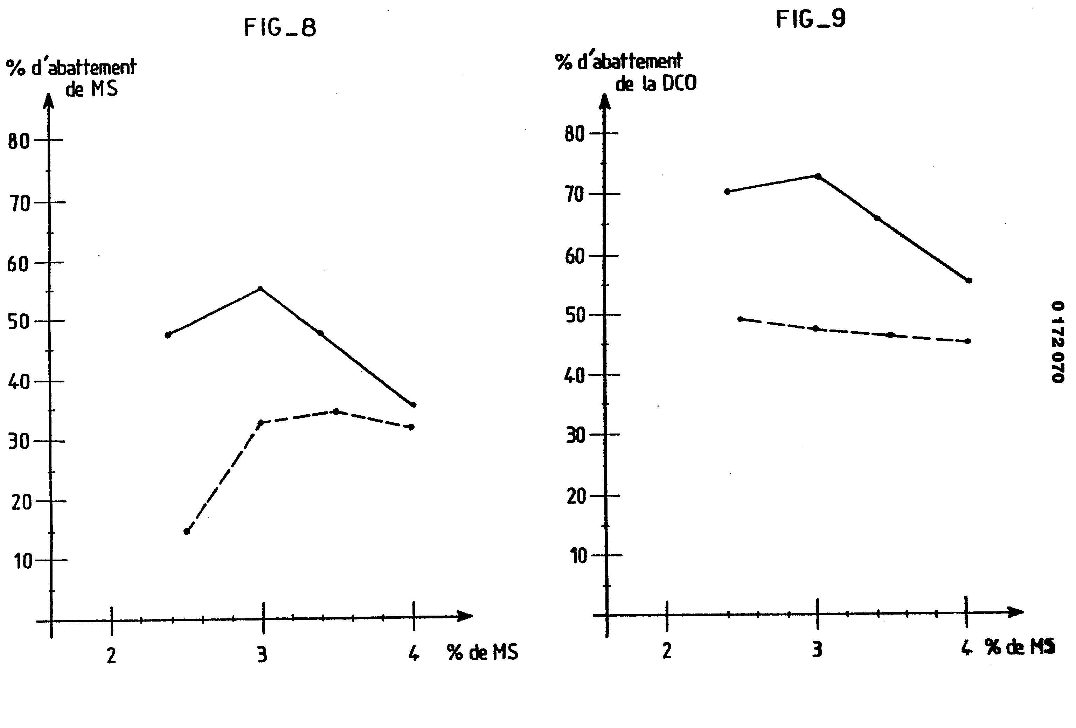

Dans les graphiques des figures 8 à 12, les courbes en trait plein représentent les résultats obtenus avec le digesteur de la figure 1, tandis que les courbes en tirets représentent les résultats obtenus avec un digesteur de l'art antérieur, dit « infiniment mélangé ».

A la figure 8, on a porté en ordonnées le pourcentage d'abattement de MS dans l'effluent digéré évacué par trop-plein par le conduit 9, en fonction du pourcentage de matière solide du lisier introduit dans le digesteur par le conduit 7. On constate une augmentation sensible de l'abattement de la MS, surtout pour des taux de MS allant de 2 à 3 %. Pour un taux de MS de 3 % dans un lisier de porc, le gain d'épuration sur la MS est de 65 %.

Au graphique de la figure 9, on a porté en ordonnées l'abattement de DCO sur la matière digérée évacuée par le conduit 9, en fonction du pourcentage de matière solide du lisier entrant par le conduit 7. Pour un taux de MS de 3 % dans un lisier de porc, le gain d'épuration sur la DCO est de 40 %.

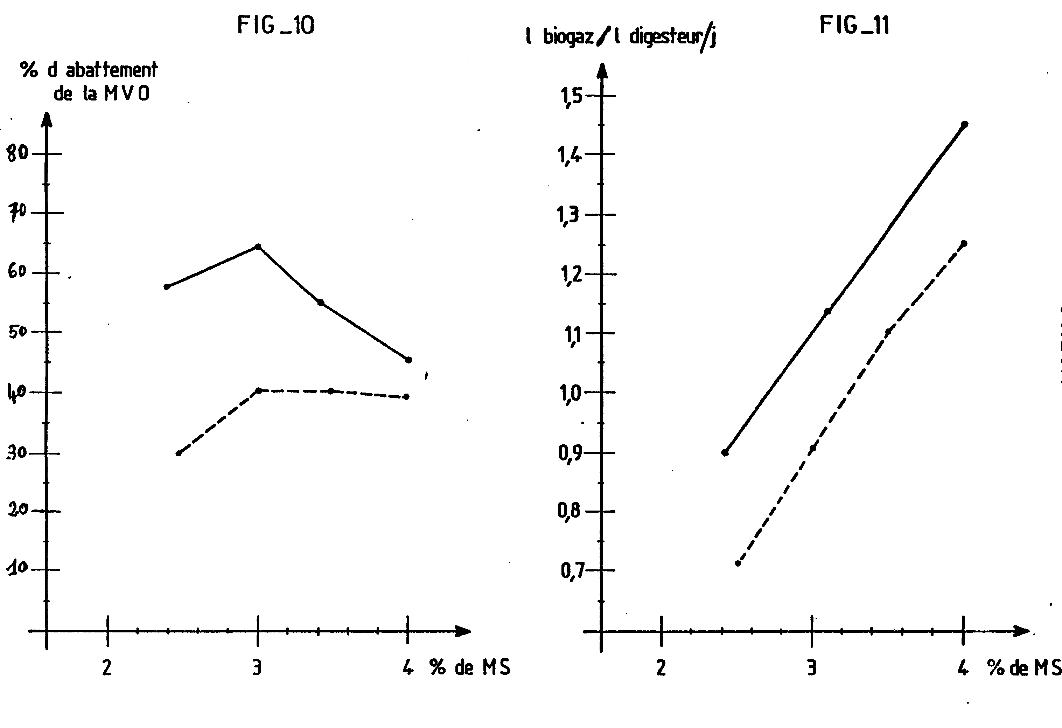

A la figure 10, on a porté en ordonnées le pourcentage d'abattement de la MVO de la matière recueillie à la sortie du conduit 9, en fonction du pourcentage de matière solide du lisier introduit par le conduit 7. Pour un taux de MS de 3 %, dans un lisier de porc, le gain d'épuration sur la MVO est de 54 %.

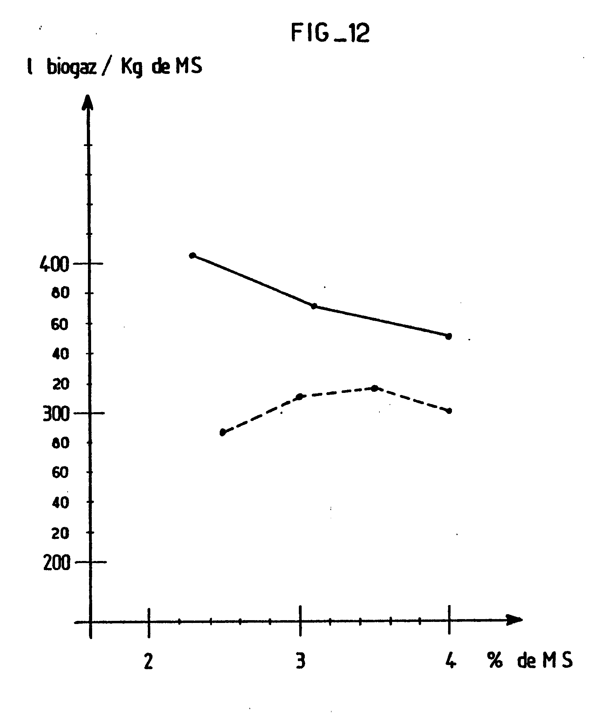

Au graphique de la figure 11, on a porté en ordonnées le nombre de litres de biogaz par litre de digesteur et par jour, recueillis par le conduit 12 en fonction du pourcentage de matière solide du lisier introduit par le conduit 7. La productivité en biogaz augmente avec le taux de matière solide. Pour un taux de matière solide de 3 % dans un lisier de porc, le gain de productivité est de 24 %.

Des analyses ont montré que la teneur en méthane est d'environ 70 %, sans variation par rapport aux procédés de digestion antérieurs.

Pour un taux de matière solide de 3 % dans un lisier de porc, le gain de rendement biologique est de 21 %. Il est encore plus important pour des taux de matière solide inférieurs à 3 % (figure 12).

Après 52 semaines de fonctionnement du digesteur, on l'arrête et on en examine l'intérieur, en procédant de la manière suivante :

Au lieu d'effectuer un chargement à l'instant prévu, on enlève le couvercle 2 supérieur et le tuyau 7 d'alimentation qui fait corps avec lui. L'enlèvement de ce tuyau entraînant une certaine perturbation, on laisse reposer pendant 6 heures. 30 heures plus tard, on examine visuellement le contenu du réacteur en se plaçant au-dessus de celui-ci. Il apparaît une fraction liquide, surnageant de lisier avec une légère mousse en surface. On enlève alors 3 fractions liquides que l'on numérote 1, 2 et 3, puis les fractions de matière décantée se trouvant dans les compartiments 18, 19, 20 et 21 respectivement.

Les volumes, les teneurs en matière sèche et les poids de chaque fraction sont donnés au tableau 4 suivant.

Tableau 4

| Fraction | Volume l | Teneur en MS en g/l | Poids en g |
|---|---|---|---|
| 1 | 1,523 | 64,4 | 98,1 |
| 2 | 0,500 | 78,8 | 39,4 |
| 3 | 1,000 | 108,8 | 108,8 |
| 4 | 1,080 | 218,7 | 236,2 |
| 5 | 0,807 | 366,8 | 296,0 |
| 6 | 0,474 | 243,5 | 115,4 |
| 7 | 0,615 | 275,8 | 169,9 |
| | 6,000 | | 1 063,8 |

# 0 172 070

Le tableau 4 fait nettement ressortir l'influence du compartiment 19 pour améliorer la digestion ; ce compartiment sert à stocker une masse relativement importante de la suspension dont la teneur en matière solide doit encore être diminuée par fermentation, avant de sortir du digesteur.

On a calculé que, environ 6 552 grammes (18 grammes × 52 semaines) sont entrés dans le réacteur en un an. Chaque jour, 9 grammes sont sortis du réacteur, soit 3 276 grammes/an. Il en résulte que 3 276 grammes ont disparu par fermentation anaérobie ou ont été stockés dans les compartiments du réacteur. Le tableau 4 montre que 1 063,8 grammes ont été stockés. Il en résulte que 2 212,2 grammes ont disparu par fermentation anaérobie.

On a étudié également la répartition par taille des particules solides dans chaque compartiment (fractions appelées ci-dessus 4, 5, 6, 7). Cette répartition est donnée au tableau 5 ci-dessous.

## Tableau 5

### Evolution de la taille des particules solides dans chaque compartiment

| R+ ⟶ 1,650 | | 0,840 | | 0,417 | | 0,347 ⟶ 0 | |
| Fraction | 1,168 | | 0,580 | | 0,246 | | |
|---|---|---|---|---|---|---|---|
| 4 | 6,98 | 14,88 | 28,34 | 7,12 | 8,08 | 4,75 | 3,93 | 25,92 |
| 5 | 1,95 | 3,35 | 18,83 | 18,33 | 17,11 | 7,87 | 5,43 | 27,13 |
| 6 | 1,52 | 3,44 | 16,77 | 13,76 | 12,20 | 7,13 | 11,81 | 33,37 |
| 7 | 0,55 | 1,36 | 6,35 | 14,62 | 19,12 | 14,42 | 7,52 | 36,06 |

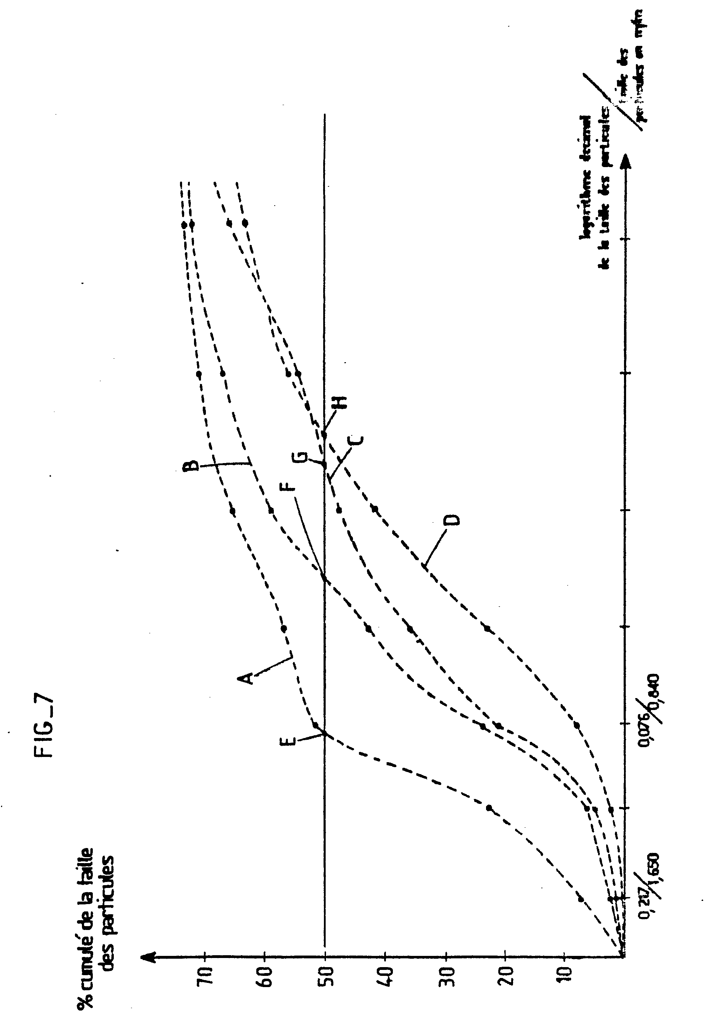

La première colonne de ce tableau donne les numéros des fractions, tandis que la ligne d'en-tête donne les limites de dimensions de particules. Dans chaque case on indique le pourcentage de particules ayant une taille comprise dans les limites indiquées pour la fraction considérée. C'est ainsi que la première case indique que, dans la fraction 4, 6,98 % des particules ont une dimension supérieure à 1,65 mm. On constate que la taille des particules tend à diminuer au fur et à mesure que l'on passe de la fraction 4 à la fraction 7, et que le pic de la distribution des particules dans chaque compartiment se déplace lui aussi vers les tailles de particules petites, au fur et à mesure que l'on passe de la fraction 4 à la fraction 7, le pic de distribution correspondant aux particules d'une dimension inférieure à 0,147 mm étant exclu. Au graphique de la figure 7, on a porté en ordonnées le pourcentage cumulé de particules pour chaque compartiment et en abscisses le logarithme décimal de la taille des particules solides. On a fait suivre ce logarithme décimal de la taille des particules elles-mêmes en millimètres. La courbe A donne la répartition dans le compartiment 18, la courbe B dans le compartiment 19, la courbe C dans le compartiment 20, et la courbe D dans le compartiment 21. Ce graphique fait apparaître de manière nette l'influence déterminante des compartiments 19 et 20, par rapport aux compartiments 18 et 21. On voit, en effet, que dans le compartiment 18 (courbe A), environ 50 % des particules ont encore une dimension supérieure à 0,84 mm. (Point È de la courbe A). Dans le compartiment 19, donc sur la courbe B, 50 % des particules ont une dimension comprise entre 0,58 et 0,417 mm, le gain en dimension de particules correspondant au segment de droite EF. Le passage de la courbe B à la courbe C entraîne aussi un gain correspondant au segment FG. En revanche, le passage de la courbe C à la courbe D n'entraîne qu'un gain correspondant au segment GH et pour une certaine finesse de particules, n'entraîne plus de gain du tout.

## Exemples 2 et 3

Ces exemples sont destinés à montrer l'influence du temps de rétention défini comme étant le rapport entre le volume du digesteur et le débit d'alimentation de celui-ci. Du point de vue industriel, l'intérêt est, toutes choses égales par ailleurs, de pouvoir réduire le volume des digesteurs. C'est dans cette optique, que les temps de rétention ont été réduits de 10 jours à 7,5 jours et à 5 jours.

## Exemple 2

Temps de rétention 7,5 jours.

Les conditions de démarrage de l'essai sont identiques à celles décrites à l'exemple 1. Après

8

remplissage du digesteur et une période de quatre jours sans alimentation, on introduit pendant une semaine 400 ml/j de lisier frais, puis 600 ml/j la semaine suivante. Le débit d'alimentation après cette période d'augmentation progressive de la charge, est maintenu à 800 ml/j durant 30 semaines d'expérimentation, au cours desquelles seules les caractéristiques du lisier seront amenées à varier.

Les analyses et relevés sont effectués selon la méthodologie présentée dans le brevet. Les caractéristiques du lisier étudié et les resultats obtenus sont consignés dans les tableaux 6, 7 et 8.

### Exemple 3

Temps de rétention 5 jours.

Pour cette expérimentation l'augmentation de charge a été effectuée de manière identique à l'exemple 2, une période d'alimentation à 800 ml/j d'une semaine supplémentaire étant nécessaire avant d'établir le débit d'alimentation à 1 200 ml/j pour 30 semaines d'expérimentation.

Les résultats sont présentés tableaux 9, 10 et 11.

Pour une porcherie produisant 20 $m^3$ de lisier à 3,5 % de MS par jour, la production de biogaz attendue par digestion anaérobie de ce substrat dans un digesteur à rétention solide avec un temps de séjour du produit de 7,5/j est de 300 $m^3$ de biogaz/j à 69 % de $CH_4$.

Le produit digéré se caractérise par une DCO inférieure à la DCO initiale de 60 à 65 %.

Le rendement biologique est de 420 l de biogaz/kg MS soit 40 % de plus qu'avec un système de digesteur infiniment mélangé.

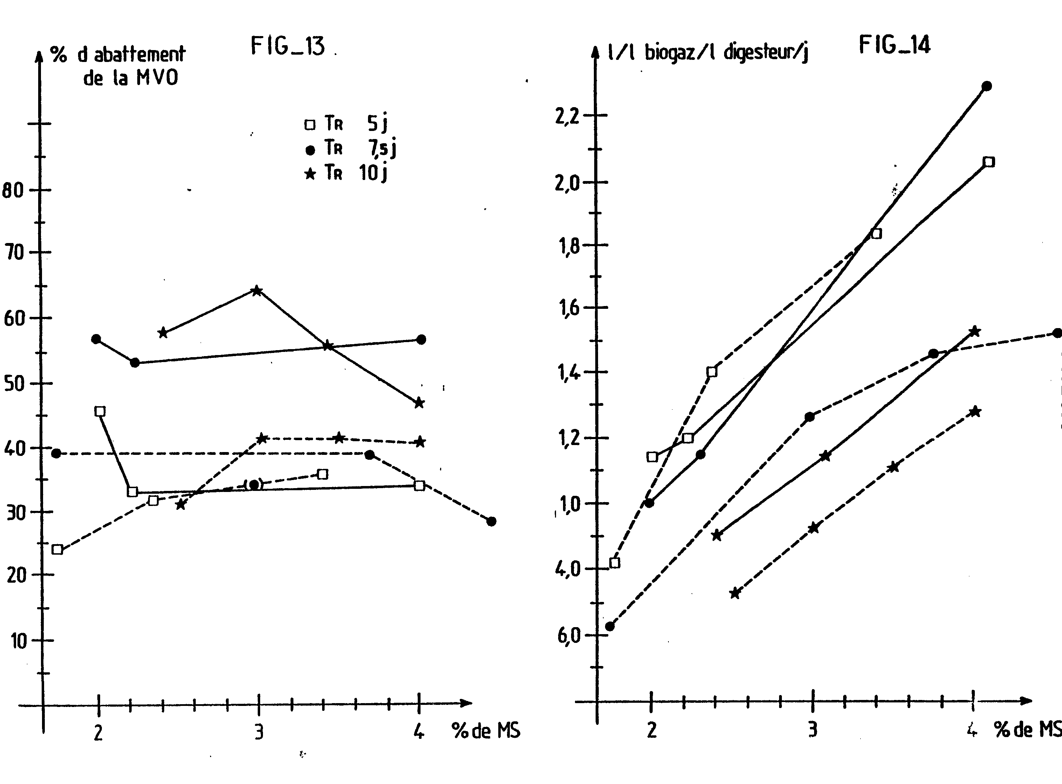

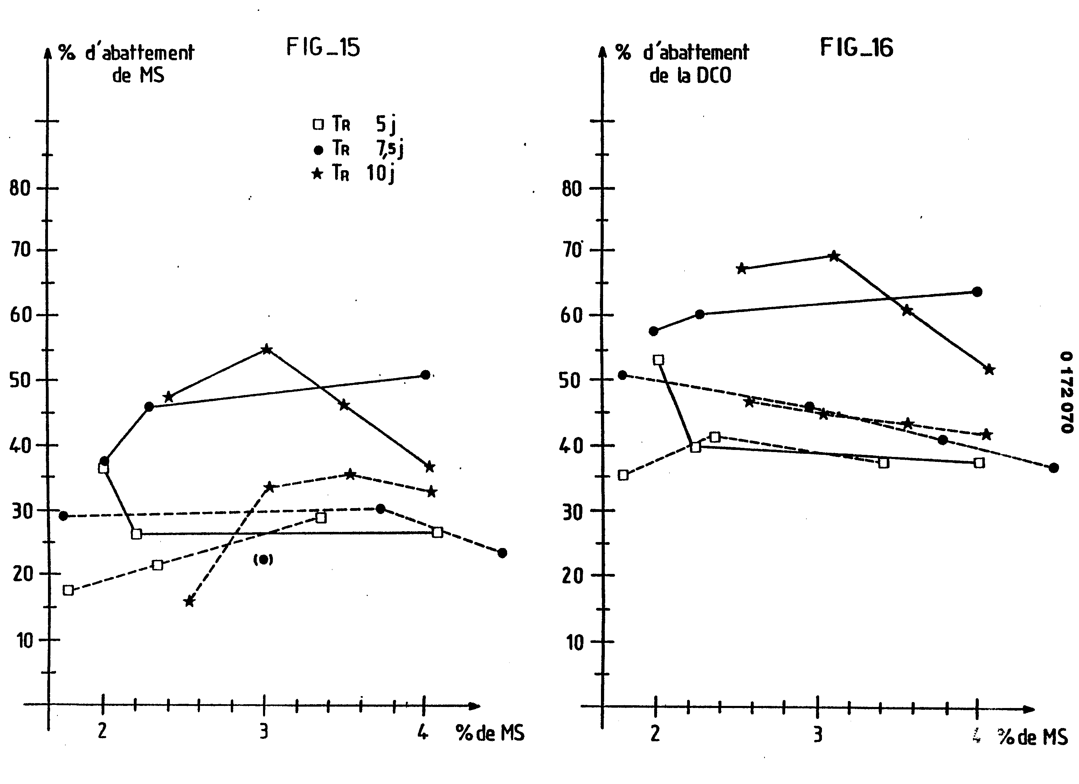

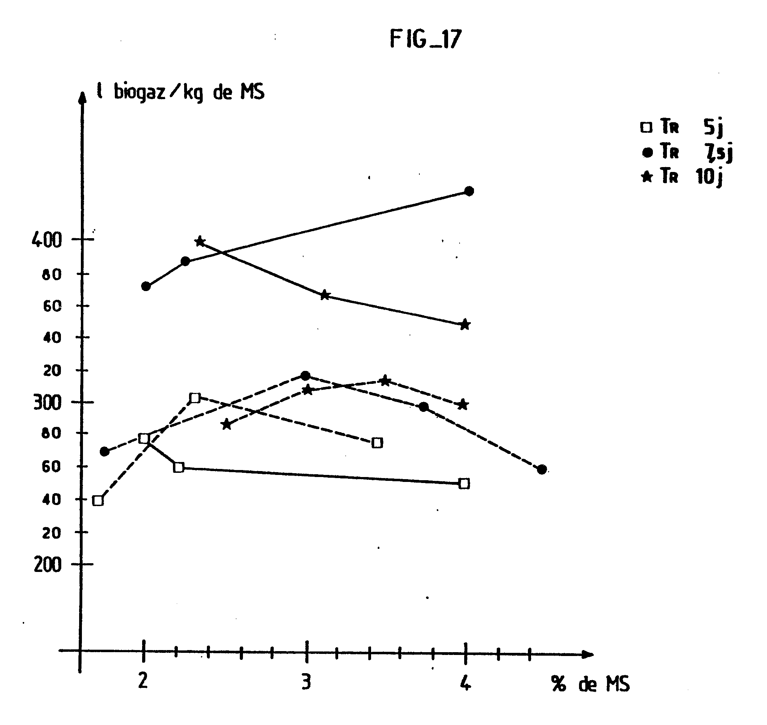

Les courbes en trait plein des figures 13 à 17 représentent les résultats obtenus avec un digesteur à rétention de solides pour un temps de séjour de 10 jours (symbole ★), 7,5 jours (symbole ●), et 5 jours (symbole □).

Les courbes en tirets représentent les résultats obtenus avec un digesteur du type infiniment mélangé pour un temps de séjour de 10 jours (symbole ★), 7,5 jours (symbole ●), et 5 jours (symbole □).

Les conclusions à tirer sont les suivantes :

1) Pour des temps de rétention de 10 jours et de 7,5 jours, les résultats obtenus par le procédé suivant l'invention sont meilleurs que ceux obtenus par le procédé antérieur dit « infiniment mélangé ».

2) Il y a possibilité d'optimiser le temps de rétention du lisier dans le digesteur, en fonction du taux de sa MS.

Par exemple, si l'on retient comme critère l'abattement de DCO, il convient de retenir un temps de rétention de 10 jours pour un taux de MS plus petit ou égal à 3,5 %, un temps de rétention de 7,5 jours pour un taux de MS supérieur à cette limite.

3) On peut en déduire qu'en tout cas le procédé suivant l'invention permet un abattement de DCO de 28 % supérieur à celui obtenu par le procédé du digesteur infiniment mélangé, quelle que soit la teneur en MS du lisier de porc, comprise entre 2 % et 4 %.

Le taux d'augmentation de la production de biogaz (1 biogaz/kg de MS) est également, pour toutes teneurs en MS du lisier comprise dans le même intervalle (2 % à 4 %), toujours supérieur ou égal à 25 % (395 l minimum contre 315 l maximum). Le pourcentage est encore amélioré, pour un temps de rétention intermédiaire entre 7,5 jours et 10 jours (par exemple 8 3/4 jours).

(Voir tableaux page 10 et s.)

Résultats : temps de rétention 7,5 jours

Tableau 6 : Epuration sur le produit brut

| | 1 | | 2 | | 3 | |
|---|---|---|---|---|---|---|
| | 1,89 %≤ MS ≤ 2,05 % | | 2,18 %≤ MS ≤ 2,36 % | | 3,73 %≤ MS ≤ 4,48 % | |
| $\overline{MS}$ % | 2,00 | σ : 0,06 | 2,23 | σ : 0,07 | 3,97 | σ : 0,21 |
| $\overline{MV}$ % | 1,25 | σ : 0,06 | 1,59 | σ : 0,06 | 2,77 | σ : 0,23 |
| $\overline{DCO}$ $mgO_2/l$ | 21050 | σ : 1060 | 24611 | σ : 1385 | 49535 | σ : 2245 |
| $\overline{\Delta MS}$ % | 36,6 | σ : 15,5 | 44,5 | σ : 15,7 | 49,3 | σ : 7,8 |
| $\overline{\Delta MV}$ % | 55,6 | σ : 17,7 | 52,2 | σ : 14,8 | 56,7 | σ : 8,5 |
| $\overline{\Delta DCO}$% | 57,2 | σ : 10,7 | 59,8 | σ : 8,6 | 63,8 | σ : 4,3 |

0 172 070

Tableau 7 : Epuration sur le surnageant de centrifugation

| | 1 | | 2 | | 3 | |
|---|---|---|---|---|---|---|
| $\overline{MS}_S$ % | 0,97 | σ : 0,06 | 0,59 | σ : 0,01 | 1,30 | σ : 0,06 |
| $\overline{MV}_S$ % | 0,49 | σ : 0,05 | 0,34 | σ : 0,01 | 0,73 | σ : 0,06 |
| $\overline{\Delta MS}_S$ % | 28,9 | σ : 2,6 | 33,3 | σ : 1,9 | 34,0 | σ : 5,0 |
| $\overline{\Delta MV}_S$ % | 51,1 | σ : 4,2 | 48,6 | σ : 3,3 | 46,3 | σ : 7,7 |

0 172 070

Tableau 8 : Production de biogaz et rendements biologiques

| | 1 | | 2 | | 3 | |
|---|---|---|---|---|---|---|
| $\overline{MS}$ % | 2,00 | $\sigma$ : 0,06 | 2,23 | $\sigma$ : 0,07 | 3,97 | $\sigma$ : 0,21 |
| $\overline{MV}$ % | 1,25 | $\sigma$ : 0,06 | 1,59 | $\sigma$ : 0,06 | 2,77 | $\sigma$ : 0,23 |
| $\overline{DCO}$ $mgO_2/l$ | 21050 | $\sigma$ : 1060 | 24611 | $\sigma$ : 1385 | 49535 | $\sigma$ : 2245 |
| $\overline{RT}$ l biogaz/l dig/j | 0,98 | $\sigma$ : 0,04 | 1,14 | $\sigma$ : 0,10 | 2,28 | $\sigma$ : 0,15 |
| % $\overline{CH_4}$ | 69,3 | $\sigma$ : 3,9 | 68,2 | $\sigma$ : 3,1 | 68,5 | $\sigma$ : 3,6 |

Tableau 8 (Suite) : Production de biogaz et rendements biologiques

| | 1 | 2 | 3 |
|---|---|---|---|
| $\overline{RB}$ 1 $CH_4$ /kg MS | 255 | 261 | 296 |
| 1 biogaz/kg MS | 368  σ : 21 | 383  σ : 45 | 432  σ : 29 |
| $\overline{RB}$ 1 $CH_4$ /kg MV | 409 | 368 | 415 |
| 1 biogaz/kg MV | 590  σ : 45 | 540  σ : 63 | 606  σ : 42 |
| $\overline{RB}$ 1 $CH_4$ /kg DCO | 244 | 237 | 237 |
| 1 biogaz/kg DCO | 352  σ : 34 | 347  σ : 25 | 346  σ : 27 |

Résultats : temps de rétention 5 jours

Tableau 9 : Epuration sur le produit brut

|  | 1 | 2 | 3 |
|---|---|---|---|
|  | $1,89\ \% \leqslant MS \leqslant 2,05\ \%$ | $2,18\ \% \leqslant MS \leqslant 2,36\ \%$ | $3,73\ \% \leqslant MS \leqslant 4,48\ \%$ |
| $\overline{MS}$ % | 2,00 $\quad$ $\sigma$ : 0,06 | 2,23 $\quad$ $\sigma$ : 0,07 | 3,97 $\quad$ $\sigma$ : 0,21 |
| $\overline{MV}$ % | 1,25 $\quad$ $\sigma$ : 0,06 | 1,59 $\quad$ $\sigma$ : 0,06 | 2,77 $\quad$ $\sigma$ : 0,23 |
| $\overline{DCO}$ $mgO_2/1$ | 21050 $\quad$ $\sigma$ : 1060 | 24611 $\quad$ $\sigma$ : 1385 | 49535 $\quad$ $\sigma$ : 2245 |
| $\overline{\Delta MS}$ % | 35,4 $\quad$ $\sigma$ : 2,2 | 25,5 $\quad$ $\sigma$ : 3,7 | 28,3 $\quad$ $\sigma$ : 9,4 |
| $\overline{\Delta MV}$ % | 45,3 $\quad$ $\sigma$ : 2,9 | 32,0 $\quad$ $\sigma$ : 3,3 | 32,2 $\quad$ $\sigma$ : 10,4 |
| $\overline{\Delta DCO}$ % | 52,1 $\quad$ $\sigma$ : 2,1 | 39,7 $\quad$ $\sigma$ : 5,7 | 37,6 $\quad$ $\sigma$ : 8,0 |

Tableau 10 : Epuration sur le surnageant de centrifugation

| | | 1 | | 2 | | 3 | |
|---|---|---|---|---|---|---|---|
| $\overline{MS}_s$ | % | 0,97 | $\sigma$ : 0,06 | 0,59 | $\sigma$ : 0,01 | 1,30 | $\sigma$ : 0,06 |
| $\overline{MV}_s$ | % | 0,49 | $\sigma$ : 0,05 | 0,34 | $\sigma$ : 0,01 | 0,73 | $\sigma$ : 0,06 |
| $\overline{\Delta MS}_s$ | % | 27,3 | $\sigma$ : 2,7 | 29,5 | $\sigma$ : 1,9 | 20,0 | $\sigma$ : 3,6 |
| $\overline{\Delta MV}_s$ | % | 42,2 | $\sigma$ : 3,1 | 42,9 | $\sigma$ : 3,4 | 20,7 | $\sigma$ : 4,3 |

0 172 070

Tableau 11 : Production de biogaz et rendements biologiques

| | 1 | | 2 | | 3 | |
|---|---|---|---|---|---|---|
| $\overline{MS}$ % | 2,00 | $\sigma$ : 0,06 | 2,23 | $\sigma$ : 0,07 | 3,97 | $\sigma$ : 0,21 |
| $\overline{MV}$ % | 1,25 | $\sigma$ : 0,06 | 1,59 | $\sigma$ : 0,06 | 2,77 | $\sigma$ : 0,23 |
| $\overline{DCO}$ $mgO_2/l$ | 21050 | $\sigma$ : 1060 | 24611 | $\sigma$ : 1385 | 49535 | $\sigma$ : 2245 |
| $\overline{RT}$ lbiogaz.1diq/j | 1,11 | $\sigma$ : 0,04 | 1,17 | $\sigma$ : 0,13 | 2,04 | $\sigma$ : 0,17 |
| $\overline{CH_4}$ % | 67,7 | $\sigma$ : 2,5 | 69,0 | $\sigma$ : 4,4 | 69,9 | $\sigma$ : 3,9 |
| $\overline{RB}$ l $CH_4$/kg MS | 188 | | 181 | | 180 | |

Tableau 11 (suite) : Production de biogaz et rendements biologiques

| | 1 | 2 | 3 |
|---|---|---|---|
| l biogaz/kg MS | 278 $\quad$ $\sigma$ : 10 | 262 $\quad$ $\sigma$ : 34 | 257 $\quad$ $\sigma$ : 28 |
| l $CH_4$/kg MV $\overline{RB}$ | 301 | 255 | 252 |
| l biogaz/kg MV | 444 $\quad$ $\sigma$ : 23 | 370 $\quad$ $\sigma$ : 49 | 361 $\quad$ $\sigma$ : 41 |
| l $CH_4$/kg DCO $\overline{RB}$ | 179 | 164 | 144 |
| l biogaz/kg DCO | 264 $\quad$ $\sigma$ : 21 | 238 $\quad$ $\sigma$ : 19 | 206 $\quad$ $\sigma$ : 22 |

0 172 070

**Revendications**

1. Digesteur formé d'une enceinte (1), d'un conduit d'entrée (7) de la matière à digérer débouchant vers le bas de l'enceinte (1), d'un conduit de sortie (9) de la matière digérée débouchant dans l'enceinte (1) à un niveau plus élevé que celui de l'embouchure du conduit d'entrée (7) et à distance de celle-ci suivant une direction horizontale, d'une conduite de sortie (12) du gaz débouchant au sommet de l'enceinte (1), et des moyens de thermostatisation (4) de l'enceinte (1), caractérisé par une première cloison (15) issue du fond (11) de l'enceinte (1) et interposée, suivant ladite direction horizontale, entre les embouchures des conduits d'entrée et de sortie (7,9) en subdivisant l'enceinte (1) en un premier compartiment (18) et en une chambre ne communiquant entre eux qu'au-dessus du bord libre (22) de la cloison (18), qui est à un niveau intermédiaire entre les deux embouchures, et par au moins une deuxième cloison (16) issue du fond (11) de l'enceinte (1) et interposée, suivant ladite direction horizontale, entre la première cloison (15) et l'embouchure du conduit de sortie (9), en subdivisant la chambre en deuxième et troisième compartiments (19, 20) ne communiquant entre eux qu'au-dessus du bord libre (23) de la deuxième cloison (16) qui est à un niveau inférieur à celui (22) de la première cloison (15).

2. Digesteur suivant la revendication 1, caractérisé en ce que le bord libre (22) de la première cloison (15) est à un niveau supérieur à la demi-hauteur de l'enceinte (1) et le bord libre (23) de la deuxième cloison (16) est à un niveau compris entre les 2/3 et le 1/3 de la hauteur de l'enceinte (1).

3. Digesteur, suivant l'une des revendications 1 ou 2, caractérisé en ce que le volume du compartiment (18) délimité entre la paroi de l'enceinte (1) et la première cloison (15) représente de 1,1 à 1,4 fois celui du compartiment (19) délimité entre la première cloison (15) et la deuxième cloison (16).

4. Digesteur, suivant l'une des revendications 1 à 3, caractérisé par une troisième cloison (17) moins haute que la deuxième (16) et subdivisant le troisième compartiment.

5. Digesteur, suivant la revendication 4, caractérisé en ce que le bord libre (24 de la troisième cloison (17) est à un niveau compris entre les 2/5 et le 1/4 de la hauteur de l'enceinte (1).

6. Digesteur suivant la revendication 4 ou 5, caractérisé en ce que le volume du compartiment (19) délimité entre la première cloison (15) et la deuxième cloison (16) représente 2,5 à 1,5 fois celui du compartiment (20) délimité entre la deuxième cloison (16) et la troisième cloison (17).

7. Digesteur suivant l'une des revendications 1 à 6, caractérisé en ce que ladite direction horizontale est circulaire, l'enceinte (1) est cylindrique et les bords (22, 23, 24) des cloisons s'étendent suivant des rayons de la section droite de l'enceinte (1).

8. Procédé de digestion anaérobie de matières organiques, qui consiste à charger périodiquement un digesteur, dans lequel on été introduites au préalable des bactéries de fermentation des matières organiques, une suspension de matières organiques, caractérisé en ce que le digesteur est tel que défini aux revendications 1 à 7 et on laisse le contenu du digesteur se décanter dans les intervalles de temps entre les changements.

9. Procédé suivant la revendication 8, caractérisé en ce que le volume de la suspension chargé à chaque chargement représente de 1/3 à 2/3 environ du volume du premier compartiment.

10. Procédé suivant l'une des revendications 8 ou 9, caractérisé en ce que la durée d'un intervalle de temps entre deux chargements est comprise entre 2 h et 24 h environ.

11. Procédé selon l'une des revendications 7 à 10, caractérisé par un temps de rétention au moins égal à 10 jours pour une teneur en matière solide inférieure ou égale à 3,5 % et par un temps de rétention de 7,5 à 8,75 jours pour une teneur en matière solide supérieure à 3,5 %.

**Claims**

1. Digester comprising an enclosure (1), an inlet duct (7) for the material to be digested which opens towards the bottom of the enclosure (1), an outlet duct (9) for the digested material which opens in the enclosure (1) at a level higher than the opening of the inlet duct (7) and at a distance therefrom in a horizontal direction, an outlet duct (12) for gas which opens from the top of the enclosure (1), and heat control means (4) for the enclosure (1), characterised by a first partition (15) running from the bottom (11) of the enclosure (1) which is interposed, in the said horizontal direction, between the mouths of the inlet and outlet ducts (7,9), thus dividing the enclosure (1) into a first compartment (18) and a chamber, which only communicate with one another above the free edge (22) of the partition (15), which is at a level intermediate between the two mouths, and by at least one second partition (16) running from the bottom (11) of the enclosure (1) which is interposed, in the said horizontal direction, between the first partition (15) and the mouth of the outlet duct (9), thus sub-dividing the chamber into second and third compartments (19, 20) which only communicate with one another above the free edge (23) of the second partition (16), which is at a lower level than the free edge (22) of the first partition (15).

2. Digester according to claim 1, characterised in that the free edge (22) of the first partition (15) is at a level higher than half the height of the enclosure (1) and the free edge (23) of the second partition (15) is at a level between two thirds and one third of the height of the enclosure (1).

3. Digester according to either of claims 1 and 2, characterized in that the volume of the compartment (18) defined between the wall of the enclosure (1) and the first partition (15) is equal to 1.1 to 1.4 times that of the compartment (19) which is defined between the first partition (15) and the second partition (16).

4. Digester according to one of claims 1 to 3, characterized by a third partition (17) which is less high than the second partition (16) and which sub-divides the third compartment.

5. Digester according to claim 4, characterised in the free edge (24) of the third partition (17) is at level between two fifths and one quarter of the height of the enclosure (1).

6. Digester according to claim 4 or 5, characterised in that the volume of the compartment (19) defined between the first partition (15) and the second partition (16) is equal to 2.5 to 1.5 times that of the compartment (20) which is defined between the second partition (16) and the third partition (17).

7. Digester according to one of claims 1 to 6, characterised in that the said horizontal direction is circular, the enclosure (1) is circular and the edges (22, 23, 24) of the partitions extend along radii of the cross-section of the enclosure (1).

8. Process for the anaerobic digestion of organic materials which consists in periodically loading a digester, into which bacteria for fermenting organic materials have previously been introduced, with a suspension of organic materials, characterised in that the digester is as defined in claims 1 to 7 and the contents of the digester are allowed to decant in the intervals between the loadings.

9. Process according to claim 8, characterised in that the volume of the suspension loaded at each loading represents approximately 1/3 to 2/3 of the volume of the first compartment.

10. Process according to either of claims 8 and 9, characterising that the length of an interval between two loadings is between approximately 2 and 24 hours.

11. Process according to one of claims 7 to 10, characterised by a retention time of at least 10 days for a solid matter content equal to or less than 3.5 % and by a retention time of 7.5 to 8.75 days for a solid matter content greater than 3.5 %.

## Patentansprüche

1. Faulbehälter, mit einem Kessel (1), einer Zuleitung (7) für Faulgut, die im unteren Teil des Kessels (1) mündet, einer Ableitung (9) für ausgefaultes Gut, die im Kessel (1) auf einem höheren Niveau als die Mündung der Zuleitung (7) und in einem waagerechten Abstand von ihr mündet, einem Ableitungsrohr (12) für Gas, das im oberen Teil des Kessels (1) mündet, und Mitteln (4) zur Temperaturregelung des Kessels (1), gekennzeichnet durch eine erste Trennwand (15), die vom Boden (11) des Kessels (1) aufragt und in der genannten waagerechten Richtung zwischen den Mündungen der Zu- und Ableitungen (7,9) angeordnet ist und dabei den Kessel (1) in eine erste Kammer (18) und einem Raum unterteilt, die untereinander nur über dem freien Rand (22) der Trennwand (15) in Verbindung stehen, der auf einem zwischen den beiden Mündungen gelegenen Niveau liegt, und wenigstens eine zweite vom Boden (11) des Kessels (1) aufragende Trennwand (16), die in der genannten waagerechten Richtung zwischen der ersten Trennwand (15) und der Mündung der Ableitung (9) angeordnet ist und dabei den Raum in eine zweite und eine dritte Kammer (19, 20) unterteilt, die untereinander nur über dem freien Rand (23) der zweiten Trennwand (16) in Verbindung stehen, welcher auf einem niedrigeren Niveau als der Rand (22) der ersten Trennwand (15) liegt.

2. Faulbehälter nach Anspruch 1, dadurch gekennzeichnet, daß der freie Rand (22) der ersten Trennwand (15) auf einem höheren Niveau als die halbe Höhe des Kessels (1) liegt, und der freie Rand (23) der zweiten Trennwand (16) auf einem Niveau zwischen zwei Dritteln und einem Drittel der Höhe des Kessels (1) liegt.

3. Faulbehälter nach Anspruch 1 oder 2, dadurch gekennzeichnet, daß das Volumen der von der Wand des Kessels (1) und der ersten Trennwand (15) begrenzten Kammer (18) das 1,1- bis 1,4 fache des Volumens der von der ersten Trennwand (15) und der zweiten Trennwand (16) begrenzten Kammer (19) beträgt.

4. Faulbehälter nach einem der Ansprüche 1 bis 3, gekennzeichnet durch eine dritte Trennwand (17), die niedriger als die zweite Trennwand (16) ist und die dritte Kammer unterteilt.

5. Faulbehälter nach Anspruch 4, dadurch gekennzeichnet, daß der freie Rand (24) der dirtten Trennwand (17) auf einem Niveau zwischen zwei Fünfteln und einem Viertel der Höhe des Kessels (1) liegt.

6. Faulbehälter nach Anspruch 4 oder 5, dadurch gekennzeichnet, daß das Volumen der von der ersten Trennwand (15) und der zweiten Trennwand (16) begrenzten Kammer (19) das 2,5- bis 1,5 fache des Volumens der von der zweiten Trennwand (16) und der dritten Trennwand (17) begrenzten Kammer (20) beträgt.

7. Faulbehälter nach einem der Ansprüche 1 bis 6, dadurch gekennzeichnet, daß die genannte waagerechte Richtung einer kreislinie folgt, der Kessel (1) zylindrisch ist, und die Ränder (22, 23, 24) der Trennwände sich in bezug auf den Querschnitt des Kessels (1) radial erstrekken.

8. Verfahren zur anaeroben Ausfaulung von organischen Stoffen, bei dem ein Faulbehälter, in den zuvor die Gärung der organischen Stoffe herbeiführende Bakterien eingesetzt worden sind, periodisch mit einer Suspension organischer Stoffe beschickt wird, dadurch gekennzeichnet, daß der Faulbehälter gemäß den Ansprüchen 1 bis 7 ausgebildet ist, und daß der Inhalt des Faulbehälters sich in den Zeitintervallen zwischen den Beschickungen absetzen kann.

9. Verfahren nach Anspruch 8, dadurch gekennzeichnet, daß das Volumen der bei jeder Beschickung eingeleiteten Suspension etwa ein Drittel bis zwei Drittel des Volumens der ersten Kammer beträgt.

10. Verfahren nach Anspruch 8 oder 9, dadurch gekennzeichnet, daß die Dauer eines Zeitintervalls zwischen zwei Beschickungen etwa zwischen 2 und 24 Stunden beträgt.

11. Verfahren nach einem der Ansprüche 7 bis 10, dadurch gekennzeichnet, daß die Verweildauer bei einem Feststoffgehalt kleiner oder gleich 3,5 % wenigstens 10 Tage und bei einem Feststoffgehalt größer als 3,5 % 7,5 bis 8,75 Tage beträgt.

FIG_1

FIG_2

FIG_3

FIG_4

FIG_5

FIG_6

FIG_7

% cumulé de la taille
des particules

70

60

50

40

30

20

10

B

A

E

F

G

H

C

D

logarithme décimal
de la taille des particules

taille des
particules en m/m

0,217/1,650    0,076/0,640

0 172 070

3

0 172 070

FIG_9

% d'abattement
de la DCO

% de MS

80 70 60 50 40 30 20 10

2   3   4

FIG_8

% d'abattement
de MS

% de MS

80 70 60 50 40 30 20 10

2   3   4

4

FIG_10

% d abattement
de la MVO

FIG_11

l biogaz / l digesteur/j

0 172 070

% de MS

% de MS

FIG _ 12

FIG_13

FIG_14

□ TR  5 j
● TR  7,5 j
★ TR 10 j

0 172 070

FIG_15

FIG_16

FIG_17